Europäisches Patentamt

European Patent Office

Office européen des brevets

(19)

(11) Publication number: **0 081 599**
**B1**

# (12) EUROPEAN PATENT SPECIFICATION

(45) Date of publication of patent specification: **24.02.88**

(21) Application number: **81110314.2**

(22) Date of filing: **10.12.81**

(51) Int. Cl.⁴: **B 22 F 9/04, B 22 F 9/12, C 09 C 1/62, C 09 C 3/04, C 23 C 14/00**

(54) Process for making metallic leafing pigments.

(43) Date of publication of application:
**22.06.83 Bulletin 83/25**

(45) Publication of the grant of the patent:
**24.02.88 Bulletin 88/08**

(84) Designated Contracting States:
**AT BE CH DE FR GB IT LI LU NL SE**

(56) References cited:
**WO-A-81/03133**
**GB-A-1 465 908**
**US-A-2 839 378**
**US-A-4 116 710**

(73) Proprietor: **Revlon, Inc.**
**767 Fifth Avenue**
**New York, N.Y.10022 (US)**

(72) Inventor: **Levine, Sol**
**Van Beuren Road**
**Morristown, New Jersey (US)**
Inventor: **Kamen, Melvin E.**
**51 Chestnut Ridge Road**
**Woodcliff Lake, New Jersey (US)**
Inventor: **De Fazio, August**
**33 Forman Lane**
**Englishtown, New Jersey (US)**
Inventor: **Cueli, Peter**
**47 Central Avenue**
**Somerville, New Jersey (US)**

(74) Representative: **Schmidt-Evers, Jürgen, Dipl.-Ing. et al**
**Patentanwälte Dipl.-Ing. H. Mitscherlich Dipl.-Ing. K. Gunschmann Dipl.-Ing. Dr.rer.nat. W. Körber Dipl.-Ing. J. Schmidt-Evers Dipl.-Ing. W. Melzer Steinsdorfstrasse 10 D-8000 München 22 (DE)**

The file contains technical information submitted after the application was filed and not included in this specification

Courier Press, Leamington Spa, England.

## Description

This invention relates to a process for producing metallic leaf pigments and to the use of these pigments in printing inks and coatings. More particularly, this invention relates to a continuous process for producing thin, bright metallic leaf pigments.

The use of metal coatings for decoration and ornamentation began several thousand years ago, but only within the last hundred years have metallic pigments become important commercially. Historically, the value of surfaces covered with gold or other metals resided not only in an aesthetic, bright, metallic finish, but such surface coatings were able to withstand the ravishes of time and weathering of the elements better than any other type of surface coating available. Due to the high cost of gold or other metals, it became difficult to produce a suitable thin leaf, and the use of metallic coatings was limited to jewelry, porcelain, chinaware and other art objects. In order to produce thin leaf or coating of metal that was but a few thousandths of an inch thick, it was necessary to begin with a ductile metal that was already hammered into extremely thin sheets. These sheets were then interleaved with animal skins and further hammered until the resultant foil was fine enough to be used. During this process the edges of the thin leaf broke off into small particles. It was then found that by mixing these small fine flakes with a drying oil, a finish could be obtained that came close to resembling a continuous sheet of metal. The artisans who worked with this type of finish prepared their metallic pigments by rubbing the finely hammered metal through a fine metal mesh.

During the mid 1800's, Bessemer produced the first practical and economical method to manufacture metallic flake pigments. This was accomplished by stamping or hammering metal sheets of appropriate brightness and then reducing the sheets into flake form which were graded and collected.

Charles Hall and Paul Herroult independently invented a practical aluminum smelting process in 1886 causing aluminum to be available in commercial quantities. Aluminum was technically adaptable to the Bessemer process but the drawback was that it formed an explosive mixture with air over a wide range of metal-air ratios.

In 1925, Everett Hall was granted a number of patents for producing a safe and superior aluminum flake pigment. This Hall process, based on a wet ball mill, carried out the size reduction of aluminum in the presence of a paint thinner containing a lubricant in solution. The lubricant was used to prevent heat cohesion of the fine flakes and the choice of lubricants determined the type of flake formed. In this process, the explosive potential from the finely powdered aluminum was minimized and a large scale commercial manufacturing process was developed. An example of the results of this invention was the paint used in 1931 to cover all structural parts of New York's George Washington Bridge.

Metallic coatings are presently obtained by using conventional aluminum flake and powder pigments dispersed in inks and then applied graphically. The metallic pigments are obtained by condensation of metallic vapors, electroplating, direct vacuum sputtering or transferred with the use of foil leaf. The coatings using conventional aluminum pigments are gray, or at best very low reflective coatings. The coatings are typically expensive, the processes difficult to control, and the processes do not lend themselves to high volume continuous coating applications. Examples of metallic coating compositions and processes for making metallic pigments are disclosed in U.S. Patent No. 2,941,894 to McAdow, U.S. Patent No. 2,839,379 also to McAdow, and U.S. Patent No. 4,116,710 to Heikel.

Heikel (US-patent No. 4,116,710=GB-patent 1 465 908) describes as one method to remove the metal coating from the substrate the use of ultrasonic vibrations but merely to assist this removal which results in metal flakes. In order to obtain flakes with smaller lateral dimensions, the released metal flake component is subjected to a cutting-type shearing force after removal of the coating metal from the substrate (GB-patent, p.2, lines 43—60). Flakes with dimensions up to 300—400 microns thereby were shredded by a sharp rotating blade. The majority of this reduced sized flakes have a largest dimension of 100 microns with a minority being within a range of 10 to 20 microns (p. 4, line 13—29). The US-patent 2,839,378 also shows that the metal film stripped from the base is to be broken up mechanically into particles (col. 4, lines 60—61). But the process of shredding mars the surface of the flakes.

Diagrams illustrating aluminum pigments production as it exists today are described in Figure 16 on p. 799 of Pigment Handbook, Volume 1 of J. Whiley & Sons, New York and Figure 5 on p. 5 of Section FA2C-1, Powder and Pigments, July 1976, Alcoa Aluminum Pigments Product Data.

Aluminum pigments, prepared as described above, have been used for some years in paints, enamels, lacquers and other coating compositions and techniques. The various grades of fineness of conventional aluminum pigments from relatively coarse particle sizes such as 250 microns (60 mesh) to about 44 microns (325 mesh).

A drawback of conventional aluminum and metallic pigments produced today is their nugget-like shape. In formulating compositions containing the conventional shaped aluminum pigments, different particle sizes and concentrations as high as 30% by weight are usual. Due to the geometric shape of the aluminum pigment particles, the particles tend to protrude from the surface of the ink or paint vehicle after drying causing a phenomenon called "dusting" which occurs when the dried coating is rubbed, thereby removing some of the metallic residue. In addition, because the pigment particles do not lie flat and are randomly distributed, the plate out is usually not uniform and requires multiple coats or applications. An additional drawback is the milling entailed in the process of size reduction in which the original brightness of the metal deteriorates and the metal takes on a gray appearance.

The present invention relates to a process as claimed in claim 1.

Figure 1 is a flow block diagram of the process for producing the metal pigments of the present invention;

Figure 2 is a schematic representation of the present process; and

Figure 3 is an enlarged view of embodiment in detail of the stripping unit.

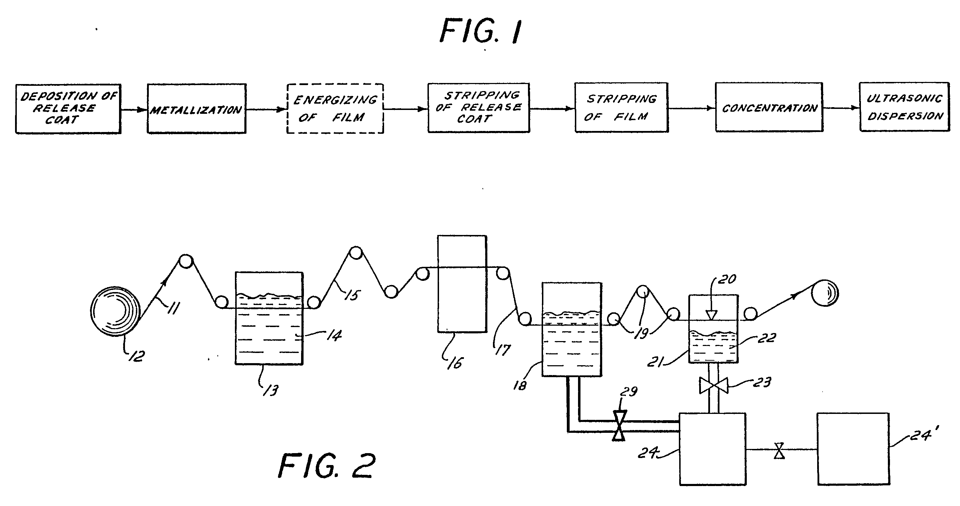

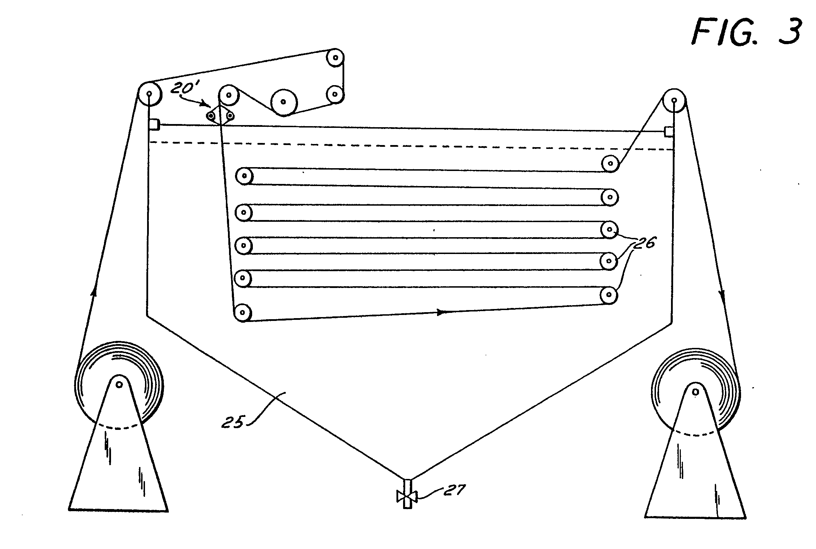

Referring now to the drawings and in particular Figure 2, a carrier sheet 11 is continuously dispensed from roll 12 and passed into vat 13 through a solution wherein a release coating 14 is applied to at least one side of carrier sheet 11. Coated carrier sheet 15 is then passed through vacuum metallizing machine 16 wherein a thin metal film is deposited on at least one side of the coated carrier sheet. The metal film-coated carrier sheet 17 is passed through stripper 18 containing a solvent in which the release coating is soluble. The stripped metallic flakes or pigments are allowed to collect in the solvent contained in stripper 18 and may then be pumped 29 to settling tanks 24, 24'. The metal film-coated carrier sheet exiting stripper 18 having the release coating solubilized may then be passed over rollers 19 and past a suitable wiper 20 in chamber 21, containing solvent 22. The wiper 20 assists in totally removing the metallic film as thin flakes and the clean carrier sheet is rewound. The metallic flakes or pigments collected in solvent 22 may then be pumped 23 to settling tanks 24, 24'. In Figure 3 there is shown an alternative embodiment of the stripping chamber, vessel 25. The metallized carrier sheet is passed through wiper 20' and around rollers 26. The metal pigments are collected at the bottom of vessel 25 and may then be pumped 27 to a settling tank.

Carrier sheet 11 may be a polyester film, for example, a polyethylene terephthalate sheet, such as Mylar®, or other suitable sheet, such as cellophane or polypropylene.

Suitable release coatings include those materials which are easily solubilized and on which the metal film may be deposited. Examples of such release coatings include polymers, such as polyvinyl chloride, polystyrene, chlorinated rubber, acrylo-nitrile-butadiene-styrene copolymer, nitrocellulose, methyl methacrylate, acrylic copolymers, fatty acids, waxes, gums, gels and mixtures thereof. Application of the release coating can be performed by solubilizing the coating material in a suitable solvent and applying with a standard continuous roll coating machine equipped to apply uniform thin coatings at commercially suitable speeds of 2,5—5 m/s (500 to 1000 feet per minute) on each side. The coated carrier sheet is preferably dried until the solvents are removed. A suitable machine is a general purpose rotogravure coater equipped with roll-to-roll unwind/wind system, such as the Two Position Rotogravure Coater and Drying Tunnel manufactured by Inta-Roto Inc. of Richmond, Virginia. The results are obtained by applying release coating in quantities of from 1,2 to 2,4 $g/m^2$ (0.75 to 1.50 lbs, per ream) preferably about 1,6 $g/m^2$ (1.0 lb. per ream (3,000 square feet)). It is essential that the release coating not be applied in amounts greater than 2,4 $g/m^2$ (1.50 lbs. per ream). Also if the coating is less than 0.25 lbs. per ream there will be insufficient release properties.

Coated carrier film 15 is passed through vacuum metallizing machine 16 and a metal film is deposited on one or both sides of the release coating. The thickness of the deposited metal film is from 35 to 45 nm and is controlled by the speed of the web and the power required for the evaporation rate. Suitable bright metals for deposition include aluminum, chromium, copper, steel, silver and gold. The metal is evaporated at rates of approximately 3.50 Ohms per square (resistance reading). This is equivalent to 35 nm on one sided coating or 7.0 Ohms or 70 nm on both sides.

The vaporization of the metal is carried out using such standard methods as induction, resistance, electronic beams and sputtering. The thickness of the deposited metal film is critical to obtain the bright particles. An extremely uniform, thin film is necessary to obtain maximum leafing properties. To obtain the desired continuous reflectiveness of the metallic particles the thickness of the film is from 35 to 45 nm. At a thickness above 45 nm the leafing properties of the particles begin to break down when put into a coating formulation. At a thickness below 35 nm, the metal particles become too delicate to separate from the resin solvent stripping system.

If desired, the carrier sheet having the thin metal film deposited thereon is stretched under tension by approximately 1 to 2% of its length causing cracks to form in the metal surface. This process as shown in Figure 1 is referred to as energizing and permits about a two-fold acceleration of the subsequent stripping process.

The metallic-coated carrier sheet 17 is then passed into a solvent tank 18 containing the solvent in which the release coating is solubilized. Suitable solvents for solubilizing the release coating include acetone, chlorinated solvents such as methylene chloride, methyl ethyl ketone, methyl isobutyl ketone, toluene, butyl acetate and the like.

The metallic film coated carrier sheet is passed through solvent tank 18 and over a series of rollers 19 and past an air knife 20 or a suitable wiper which removes loose metal particles from the carrier sheet. The air knife may be in the same chamber 18 as the solvent but usually is in a separate chamber 21, as shown in Figure 2, containing a solvent 22, which solvent may or may not be the same solvent as in tank 18. It is essential that the solvent be non-reactive with the metallic pigment. A suitable air knife can be formed from a hollow tube fitted to a source of compressed air of approximately 6,2 bar (90 PSI). Nozzles or fine holes are machined laterally and equally spaced along its length so when assembled the air jets project at a tangent to the moving carrier sheet. The air jets remove all residual metallic flakes that may remain on the film. The air knife also acts as a drying mechanism for the wetted carrier sheet thereby aiding in rewinding. In addition it may be desirable to use a vapor degreasing technique to totally remove both residual metal and release coating from the carrier sheet prior to rewinding. The vapor degreasing also cleanses any residual release coating from any remaining metal flakes. The air may be ambient, chilled or heated for optimum efficiency.

The solvent in tank 18 may be used until saturated. Moreover, the solvent may be recovered from the solution containing the coating material and the residual coating material can be reused in a subsequent coating operation, if it is properly purified.

The pigment dispersed in the solvent is either allowed to settle in stripping tank 18 or 21 or transported by pump 29 or 23 to settling tanks 24, 24' or passed through a centrifuge so that a concentrated suspension of thin bright metallic pigment is obtained. The concentration of metallic pigment in solvent is preferably about 0.045%, but in any event the concentration does not exceed 0.2% prior to centrifuging.

The metallic pigment is then broken up into particles of which preferably about 90% are from 25—50 μm in diameter. A means for reducing the pigment to the proper particle size is a sonolator which operates by ultrasonic action and thereby does not destroy the reflectivity of the bright surface of the pigment particles. A suitable ultrasonic dispersion is the Triplex Sonolator System, Model A HP, Type A, Design 150 made by the Sonic Corp. of Stratford, Connecticut.

The thin, bright metallic pigment having a diameter from 25—50 μm is then concentrated to a pigment solids of from 5 to 15%. The concentrated pigment may then be formulated into a spray lacquer or a printing ink.

It has, however, been found preferable to further concentrate the metallic pigment by first using a solvent exchange with, for example, methyl cellosolve, followed by further concentration in a centrifuge wherein the metallic solids are collected at approximately 20%. This concentrate is then made into a lacquer or printing ink formulation at a metal concentration of from 1.0—5.0% by weight of the metal. The formulation is finally passed through a means which homogenizes the formulation and brings the ultimate metallic particle size to approximately 10—20 μm. It was surprisingly found that coating formulations containing from 1—5% by weight of aluminum pigments made according to the process of this invention gave continuous mirror finishes with excellent hiding power.

The metallic film obtained in this process resembles the brilliance, reflective gloss, and hiding power of commercial metallic foils. Due to the natural orientation of the single layer leafing flake, extremely small amounts of pigments will cover very large surface areas. For example, 130 grams of the solid aluminum brilliant leafing pigment of this invention converts to 3,79 l (1 gallon) of printing ink and will yield excellent coverage of approximately 1940 m² (3,000,000 square inches) with a #300 quadrangular cell rotogravure printing cylinder. The efficiency of the lay down is due to the ability of the product of this invention to reconstruct itself.

The following examples illustrate the practice of the above-described invention.

Example 1

An aluminum pigment was made in the following manner. A release coat comprising 10% nitrocellulose in toluene was coated onto a 12,5 μm (½ mil.) thick Mylar® carrier sheet with a 200 line quad rotogravure roll on a commercial roll coater and dried, leaving a glossy film of nitrocellulose on the carrier sheet. The coated carrier sheet was then metallized on a CVC Vacuum Roll Coater applying 40±5 nm of thickness of aluminum film. This metallized, coated carrier sheet was then passed through a stripping machine and a suspension of aluminum flakes was collected having a concentration of about 0.1% by weight of aluminum flakes. Solvents used in the stripping process were composed of 50% toluene and 50% methyl ethyl ketone (MEK). The aluminum flake containing suspension was then allowed to settle out and further concentrated to about 6% solids.

Example 2

A 12,5 μm (½ mil) thick Mylar® carrier sheet was coated with a 10% nitrocellulose solution in a commercial roll coater machine using a 100 line rotogravure roller. A second coating of chlorinated rubber was subsequently applied on top of the nitrocellulose. The coated carrier sheet was then metallized with 40±5 nm of aluminum and the metallized carrier sheet stripped in a bath comprised of 25% acetone, 25% toluene, 25% MEK and 25% butyl acetate. The metal particles were then concentrated to 6% solids aluminum.

Example 3

A 12,5 μm (½ mil) thick cellophane carrier sheet was coated with an acrylic copolymer dissolved in toluene in an amount of approximately 1,6 g/m² (1.0 lb. per ream). The coated sheet was subsequently metallized with 40±5 nm of aluminum and then stripped with a solution comprising 50% toluene, 40% MEK and 10% acetone. The aluminum flakes stripped easily and were bright.

Example 4

A 12,5 μm (½ mil) thick polyester carrier sheet was coated on a commercial machine with about 1,6 g/m² (1.0 lb. per ream) per side of a release coating comprising a mixture of methyl methacrylate resin and

# 0 081 599

acrylic copolymer dispersed in 50% MEK and 50% toluene. The coated sheet was then metallized with copper about 40 nm thickness. The release coat was solubilized with methylene chloride and the thin bright copper particles were collected.

Example 5

A 12,5 µm ($\frac{1}{2}$ mil) thick polyethylene terephthalate carrier sheet was coated on both sides with approximately 2 g/m² (1.25 lbs. per ream) of nitrocellulose dispersed in toluene. The coated carrier sheet was then metallized on both sides with 40±5 nm thickness of aluminum. The metallized carrier sheet was then stripped by dissolving the release coating in a solvent which comprised 45% MEK, 45% toluene and 10% acetone. The thin aluminum particles were collected in the solvent mixture.

Example 6

A 12,5 µm ($\frac{1}{2}$ mil) thick Mylar® carrier sheet was coated with nitrocellulose at about 1,6 g/m² (1.0 lbs. per ream) per side on a commercial coating machine then metallized with approximately 40±5 nm thickness of chromium. The nitrocellulose release coating was solubilized with a solution of 50% MEK and 50% toluene and the chromium flakes were stripped from the carrier sheet.

Products obtained from Examples 1 through 6 were placed in a centrifuge and spun at 13,000 to 16,000 rpm for 5 minutes. A pigment concentration of approximately 10 to 20% by weight of pigment was obtained when the supernate was removed.

The metallic pigments of Examples 1 through 6 and spun in the centrifuge were passed through a sonolater using a gap orifice 21. At least 90% of the particles were examined and found to have peripheral dimensions ranging from 25 to about 50 µm in diameter. Of course, the thickness of the particles remained about 40±5 nm. These metallic pigments were further concentrated to approximately 10% and passed through an ultrasonic disperser and were uniformly reduced to an optimum pigment dimension of between 10 to 20 µm.

The aluminum pigments obtained were formulated into a printing ink having the following formulation:

|  | Amount |
|---|---|
| Aluminum leafing pigment (on solids basis) | 5 g |
| Nitrocellulose | 1 g |
| Stearic acid | 5 g |
| Methyl/ethyl cellosolve 60%/40% | 93.5 g |

A printing ink of this formulation using a 300 line screen roller then calendered with polished steel rollers at approximately 82°C (180°F). gave the same effect as applying hot stamping foil or aluminum foil to a laminated board.

An aluminum pigment made according to the process of this invention was made into a spray lacquer of the following formulation:

|  | Amount |
|---|---|
| Aluminum pigment | 1 g |
| Acrylic binder | 0.5 g |
| Wetting agent | 0.1 g |
| Methyl/ethyl cellosolve 60/40% | 98.4 g |
|  | 100.0 g |

3,79 l (1 gallon) of lacquer basis on this formulation was found to satisfactorily yield approximately 225—258 m² (350,000 to 400,000 square inches) of sprayed surface when using conventional spray equipment.

In order to obtain maximum coating effects and compatibility in printing, coating, lacquer and paint products, a fine particle size is required to obtain a highly reflective film. However by reducing the ultrasonic treatment it is possible to obtain a sparkle-like effect at lower concentrations of pigments.

**Claims**

1. A process for the continuous production of metallic particles comprising

— applying a release coating onto at least one side of a continuous carrier sheet;
— depositing a film 35 to 45 nanometers thick of a metal selected from the group consisting of aluminum, chromium, copper, steel, silver and gold directly onto said release coating;

5

— passing said carrier sheet, with said release coating and said metal film deposited thereon, through a first solvent which dissolves said release coating but which is non-reactive with said metal;

— removing said metal film from the carrier sheet in a particulate form to produce the metal particles substantially free of the release coating, and collecting the metal particles in a second solvent which is non-reactive with the metal;

— concentrating said metal particles in said solvent;

characterized in that said release coating is applied in an amount of 1,22 to 2,44 grams per square meter (0.00025 to 0.0005 lb/sq.ft) per side of carrier sheet and in that said metal particles are broken into particles having a size diameter of between 25 and 50 µm by ultrasonic agitation.

2. A process according to claim 1 characterized by the fact that the selected metal is aluminium.

3. A process according to claim 2, characterized by the fact that the carrier sheet is formed of polyethylene terephthalate.

4. A process according to claim 3, characterized by the fact that the release coating is selected from polyvinyl chloride, polystyrene, chlorinated rubber, acrylonitrilebutadiene-styrene copolymer, nitrocellulose, cellophane, methyl methacrylate, acrylic copolymers, fatty acids, waxes, gums, gels and mixtures thereof.

5. A process according to claim 4, characterized by the fact that the film of the metal is deposited on the release coating by vaporizing the metal and then condensing it.

6. A process according to claim 5, characterized by the fact that the carrier sheet is stretched by 1 to 2% in length prior to removing said metal film therefrom.

7. A process according to claim 5 or 6 characterized by the fact that the step of removing said metal film is carried out with an air knife.

8. A process according to claim 7, characterized by the further steps of:

further concentrating said metal pigment particles to produce a solids concentration of from 5% to 15%, and

reducing the metal particles to a particle size diameter between about 10 to 20 µm.

9. A process according to claim 1, characterized by the fact that said first solvent and said second solvent are identical to one another.

10. A process according to claim 8, characterized by the fact that the metal pigment particles are reduced by ultrasonic dispersion.


**Patentansprüche**

1. Verfahren zur kontinuierlichen Herstellung von metallischen Teilchen, umfassend

das Aufbringen eines Trennüberzugs auf wenigstens eine Seite einer kontinuierlichen Trägerschicht,

das Aufbringen eines 35 bis 45 Nanometer dicken Films von einem Metall aus der Gruppe Aluminium, Chrom, Kupfer, Stahl, Silber und Gold unmittelbar auf diesen Trennüberzug,

das Hindurchführen der Trägerschicht mit dem Trennüberzug und dem darauf niedergeschlagenen Metallfilm durch ein erstes Lösungsmittel, welches den Trennüberzug löst, aber in Bezug auf das Metall nicht reaktiv ist,

die Entfernung des Metallfilms von der Trägerschicht in einer Partikelform zur Gewinnung von Metallteilchen, die im wesentlichen frein sind von dem Trennüberzug und

das Sammeln der Metallteilchen in einem zweiten Lösungsmittel, welches gegenüber dem Metall nicht reaktiv ist sowie Konzentrieren der Metallteilchen in diesem Lösungsmittel,

dadurch gekennzeichnet,

daß der Trennüberzug aufgebracht ist in einer Menge von 1,22 bis 2,44 g/m² (0,00025 bis 0,0005 lb/sq.ft) pro Seite der Trägerschicht und daß die Metallteilchen in metallische Pigmentteilchen mit einem Teilchendurchmesser zwischen 25 und 50 µm durch Ultraschall zerkleinert werden.

2. Verfahren gemäß Anspruch 1, dadurch gekennzeichnet, daß das ausgewählte Metall aus Aluminium besteht.

3. Verfahren gemäß Anspruch 2, dadurch gekennzeichnet, daß die Trägerschicht aus Polyäthylenterephthalat gebildet ist.

4. Verfahren gemäß Anspruch 3, dadurch gekennzeichnet, daß der Trennüberzug ausgewählt ist aus der Gruppe Polyvinylchlorid, Polystyrol, Chlorkautschuk, Acrylnitril-Butadien-Styrol-Copolymere, Nitrocellulose, Cellophan, Methylmethacrylatpolymere, Acrylcopolymere, Fettsäuren, Wachse, Gummiharzen, Gelen und daren Mischungen.

5. Verfahren gemäß Anspruch 4, dadurch gekennzeichnet, daß der metallische Film auf dem Trennüberzug durch Verdampfen des Metalls und anschließendes Kondensieren niedergeschlagen ist.

6. Verfahren gemäß Anspruch 5, dadurch gekennzeichnet, daß die Trägerschicht um 1 bis 2% in der Länge gestreckt wird vor dem Entfernen des Metalls.

7. Verfahren gemäß Anspruch 5 oder 6, dadurch gekennzeichnet, daß die Entfernung des Metallfilms durch eine Luftbürste bewirkt wird.

8. Verfahren gemäß Anspruch 7, gekennzeichnet durch die weiteren Stufen des Aufkonzentrierens der metallischen Pigmentteilchen auf eine Konzentration zwischen 5 und 15% und des Verkleinerns der Metallteilchen auf einen Teilchendurchmesser zwischen 10 und 20 µm.

9. Verfahren gemäß Anspruch 1, dadurch gekennzeichnet, daß das erste Lösungsmittel zum Lösung des Trennüberzugs und das zweite Lösungsmittel untereinander identisch sind.

10. Verfahren gemäß Anspruch 8, dadurch gekennzeichnet, daß die metallischen Pigmentteilchen durch Ultraschalldispersion verkleinert werden.

**Revendications**

1. Procédé de fabrication continu de particules métalliques, consistant à:

— appliquer un enduit séparateur sur au moins une face d'une feuille support continue;
— déposer un film de 35 à 45 nanomètres d'épaisseur d'un métal choisi dans le groupe composé de l'aluminium, du chrome, du cuivre, de l'acier, de l'argent et de l'or, directement sur ledit enduit séparateur;
— faire passer ladite feuille support, avec ledit enduit séparateur et ledit film métallique déposé sur cet enduit, à travers un premier solvant qui dissout ledit enduit séparateur mais qui est non réactif avec ledit métal;
— éliminer ledit film métallique de la feuille support sous une forme particulaire pour produire les particules métalliques sensiblement exemptes de l'enduit séparateur, et recueillir les particules métalliques dans un deuxième solvant qui est non réactif avec le métal;
— concentrer lesdites particules métalliques dans ledit solvant;

procédé caractérisé en ce que ledit enduit séparateur est déposé en une quantité de 1,22 à 2,44 grammes par mètre carré (0,00025 à 0,0005 livre/pied carré) par face de la feuille support, et en ce que lesdites particules métalliques sont fragmentées en particules ayant un diamètre compris entre 25 et 50 µm par agitation par les ultrasons.

2. Procédé selon la revendication 1, caractérisé par le fait que le métal choisi est l'aluminium.

3. Procédé selon la revendication 2, caractérisé par le fait que le feuille support est formée de téréphtalate de polyéthylène.

4. Procédé selon la revendication 3, caractérisé par le fait que l'enduit séparateur est choisi entre le polychlorure de vinyle, le polystyrène, le caoutchouc chloré, le copolymère acrylonitrilebutadiène-styrène, la nitrocellulose, la cellophane, le méthacrylate de méthyle, les copolymères acryliques, les acides gras, les cires, les gommes, les gels et les mélanges de ces substances.

5. Procédé selon la revendication 4, caractérisé par le fait que le film de métal est déposé sur l'enduit séparateur par vaporisation du métal puis condensation de ce métal.

6. Procédé selon la revendication 5, caractérisé par le fait que la feuille support est étirée de 1 à 2% en longueur avant d'en séparer le film métallique.

7. Procédé selon la revendication 5 ou 6, caractérisé par le fait que le phase consistant à séparer ledit film métallique s'effectue au moyen d'une lame d'air.

8. Procédé selon la revendication 7, caractérisé par les phases suivantes consistant à:
concentrer davantage lesdites particules de pigment métallique pour produire une concentration de solides entre 5% et 15%; et
réduire les particules métalliques à un diamètre de dimension de particule entre environ 10 et 20 µm.

9. Procédé selon la revendication 1, caractérisé par le fait que ledit premier solvant et ledit deuxième solvant sont identiques entre eux.

10. Procédé selon la revendication 8, caractérisé par le fait que les particules de pigment métallique sont réduites par dispersion aux ultraons.

# FIG. I

| DEPOSITION OF RELEASE COAT | → | METALLIZATION | → | ENERGIZING OF FILM | → | STRIPPING OF RELEASE COAT | → | STRIPPING OF FILM | → | CONCENTRATION | → | ULTRASONIC DISPERSION |

# FIG. 2

0 081 599

FIG. 3

0 081 599